# EUROPEAN PATENT APPLICATION

(11) **EP 1 445 769 A1**
(43) Date of publication of application: **11.08.2004**
(21) Application number: 03002473.1
(22) Date of filing: 05.02.2003
(51) Int. Cl.: G11B 7/24, G02F 1/361, C09B 23/00, C07D 333/60

(54) **Optical layers comprising dyes based on 3-dicyanomethylidene-2,3-dihydrothiophen-1,1-dioxide derivatives**

(71) Applicant: CLARIANT INTERNATIONAL LTD., 4132 Muttenz (CH)
(72) Inventor: Metz, Hans Joachim, 64285 Darmstadt (DE); Steffanut, Pascal, 68680 Kembs Loechle (FR); Winter, Martin, 65779 Kelkheim/Ruppertshain (DE)

(57) **Abstract**

The present invention relates to blue 3-dicyanomethylidene-2,3-dihydrothiophen-1,1-dioxide based dyes which are useful as dyes in optical layers. In particular the invention relates to optical layers comprising such dyes, to a method for manufacturing such optical layers and to optical recording media comprising such optical layers.

## Description

The present invention relates to blue 3-dicyanomethylidene-2,3-dihydrothiophen-1,1-dioxide based dyes which exert high light-fastness properties and which are useful as dyes in optical layers.

Organic dyes have attracted considerable attention in the field of diode-laser optical storage. Commercial recordable compact discs (CD-R,CD-RW) and recordable digital versatile discs (DVD-R, DVD-RW) can contain, as recording layer (or so called optical layer), numerous dyes based on phthalocyanine, hemicyanine, cyanine and metallized azo structures. These dyes are suitable in certain areas in accordance with the laser wavelength criteria. Other general requirements for dyes in optical media are strong absorption, high reflectance, high recording sensitivity, low thermal conductivity as well as light and thermal stability, durability for storage and non-toxicity.

Systems for optical data recording are known in the prior art, which comprise as an absorbing compound dyes of the triphenylmethane type (JP-OS 112 793/1983), oxazine type (JP-OS 132 231/1983), cyanine type (JP-OS 112 790/1983 and 125 246/1983), phthalocyanine type (EP-A 84 729, US-PS 42 41 355, JP-OS 56 892/1983), 1,4-naphthochinone type (JP-OS 112 793/1983) and of the azo type (DE-OS 342 61 92 A1). The optical layers obtained thereby are suitable in a spectral range corresponding to the wavelength of a He-Ne-laser (633 nm).

Among this large number of "push-pull" structures already synthesised, only a few use the combination of dicyanomethylene and sulfone as acceptor groups. The use of such heterocyclic acceptors with two strong electron-withdrawing groups has first been described by *Alheim at al*. in 1996. The particular substitution pattern led to molecules with properties suitable for non-linear optics (NLO).

The construction of optical data recording media is known in the art. An optical data recording media generally comprises a substrate and a recording layer, the optical layer. Usually disks or wavers of organic polymeric materials are used as substrates. Preferred substrates are polycarbonate (PC) or polymethylmethacrylate (PMMA). The substrate has to provide an even and uniform surface of high optical quality. The optical layer is deposited thereon in a thin and uniform film of high optical quality and defined thickness. Finally, a reflective layer, e.g. aluminium, gold, copper, is deposited upon the optical layer.
Advanced optical data recording media may comprise further layers, such as protective layers, adhesive layers or additional optical layers.

To provide for a thin and uniform film of the optical layer, the material is usually deposited by spin coating, vacuum evaporation, jet coating, rolling coating, or soaking. The preferred process in industry is spin coating to form an optical layer of about 70 nm to 250 nm thickness. For the application in the spin coating process, the material of the optical layer has to be highly soluble in organic solvents.

From the German patent DE 2929001 C2 dispersion dyes of the following general formula are known, which exert blue nuances in the textile dyeing of polyester materials.

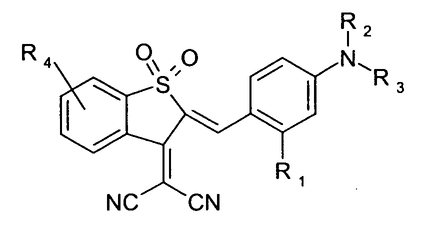

The objective of the present invention is to provide for dyes to be used in optical layers, which exert high absortivity, high recording sensitivity, high light-fastness and high thermal stability as well as low thermal conductivity.
A further objective of the present invention is to provide for dyes, which can be applied by using the spin coating process.

It now has been found that 3-dicyanomethylidene-2,3-dihydrothiophen-1,1-dioxide based dyes with particular substitution pattern provide for the above described properties required for optical layers.

The present invention therefore relates to optical layers comprising blue 3-dicyanomethylidene-2,3-dihydrothiophen-1,1-dioxide based dyes and their use in optical recording media and more particularly to optical layers comprising chromophores derived from 2-[2-(4-amino-benzylidene)-1,1-dioxo- 1,2-dihydro-1-*lambda*-6-benzo[b] thiophen-3-ylidene]-malononitrile.

The invention further relates to a Write Only Read Many (WORM) type optical recording medium capable of recording and reproducing information with radiation of red laser in a laser wavelength range of from 500 to 700 nm, which employs chromophores derived from 2-[2-(4-amino-benzylidene)-1,1-dioxo-1,2-dihydro-1-*lambda*-6-benzo[b]thiophen-3-ylidene]-malononitrile in the optical layer.

Dyes based on 3-dicyanomethylidene-2,3-dihydrothiophen-1,1-dioxide are known in the art and can be easily synthesised with high yields.

In one embodiment, the invention relates to an optical layer comprising at least one blue disperse dye of the formula (I) 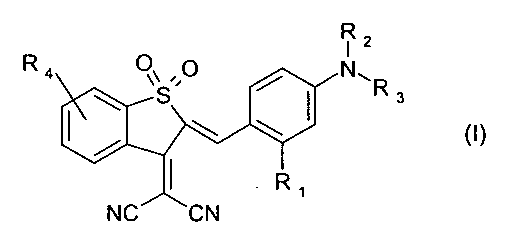 wherein
- R₁ is: selected from hydrogen, hydroxyl, C₁₋₈ alkyl, C₁₋₈ alkoxyalkyl, trifluoromethyl, carboxyl (COOH), carboxylic ester (COOR⁵) nitro (NO₂), amide (CONR⁶R⁷), sulfonate (SO₃R⁸), and halogen (-F, -Cl, -Br);
- R² and: R³ are independently selected from hydrogen, C₁₋₈ alkyl, C₁₋₈ alkoxyalkyl, cyanoalkyl, or
R² and R³ can be connected to form a pyrrolidine ring, a piperidine ring or a morpholine ring, or
R² and R³ can form a julolidine ring connecting R² and R³ to the ortho-positions at the benzene ring;
- R₄ is: selected from hydrogen, C₁₋₂ alkyl, C₁₋₂ alkoxyalkyl, nitro (NO₂), and halogen (-F, -Cl, -Br);
- R⁵,: R⁶, R⁷or R⁸ are selected from hydrogen and C₁₋₈ alkyl.

The dyes based on 2-[2-(4-amino-benzylidene)-1,1-dioxo-1,2-dihydro-1-*lambda*-6-benzo[b] thiophen-3-ylidene]-malononitrile possess the required optical characteristics for optical layers, an excellent solubility in organic solvents, an excellent light stability and a high decomposition temperature.

In a preferred embodiment, the invention relates to an optical layer comprising at least one blue disperse dye of the formula (I) 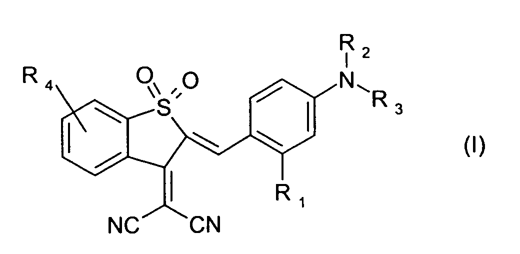 wherein
- R₁ is: selected from hydrogen, hydroxyl, C₁₋₂ alkyl, C₁₋₂ alkoxyalkyl, and halogen (-F, -Cl, -Br);
- R² and: R³ are independently selected from C₁₋₈ alkyl, C₁₋₈ alkoxyalkyl, or
R² and R³ can be connected to form a pyrrolidine ring, or
R² and R³ can form a julolidine ring connecting R² and R³ to the ortho-positions at the benzene ring;
- R₄ is: selected from hydrogen, C₁₋₂ alkyl, C₁₋₂ alkoxyalkyl, nitro (NO₂), and halogen (-F, -Cl, -Br).

More preferred are compounds of the formula (I) wherein
- R₁: is selected from Methyl, Methoxy, Chloro or Bromo;
- R² and: R³ are independently selected from C₂₋₆ alkyl, or
R² and R³ can be connected to form a pyrrolidine ring, or
R² and R³ can form a julolidine ring connecting R² and R³ to the ortho-positions at the benzene ring;
- R₄: is hydrogen.

Most preferred are compounds of the formula (I) wherein
- R₁: is methyl,
- R₂ and R₃: are n-Hexyl, and
- R₄: is hydrogen.

This most preferred compound (CAS 74239-96-6) is available from Clariant GmbH, Division PigmentsAdditives, Sulzbach am Taunus, Germany, under the trademark Foron® Brilliant Blue S-R.

### Preparation of dyes based on 3-dicyanomethylidene-2,3-dihydrothiophen-1,1-dioxide

The synthesis of blue disperse dyes according to formula (I) is generally disclosed in the US patent 4,281,115 which is incorporated herein by reference.

The blue disperse dyes according to formula (I) are obtained by condensing a compound of the general formula (A) with a compound of the general formula (B) in a 1:1 ratio.

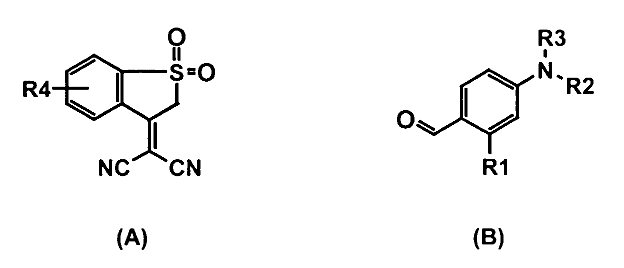

Preferably a methylene activated compound of type A is condensed with one equivalent of a 4-amino substituted benzaldehyde of type B. The solvent used in the condensation reaction is selected from, C₁₋₈ alcohols, aromatics, dimethylformamide, N-methylpyrolidone or a mixture of any of these solvents with water.

### Preparation of the optical layer

Dyes based on 3-dicyanomethylidene-2,3-dihydrothiophen-1,1-dioxide according to the invention can be directly applied from solution to a substrate to form an optical layer. The layer can be formed by spin coating, vacuum evaporation, jet coating, rolling coating, or soaking. The preferred process in industry is spin coating to form an optical layer of 70 nm to 250 nm thickness.

In one of its aspects the invention relates to a method for producing optical layers, comprising the following steps:
(a) providing a substrate
(b) dissolving a compound of formula (I) as defined above in an organic solvent to form a solution,
(c) coating the solution (b) on the substrate (a);
(d) evaporating the solvent to form a dye layer.

The organic solvent in step (b) is selected from C₁₋₈ alcohol , halogen substituted C₁₋₈ alcohols, C₁₋₈ ketone, C₁₋₈ ether, halogen substituted C₁₋₄ alkane, or amides.
The C₁₋₈ alcohols or halogen substituted C₁₋₈ alcohols are selected from methanol, ethanol, isopropanol, diacetone alcohol (DAA), 2,2,3,3-tetrafluoropropanol, trichloroethanol, 2-chloroethanol, octafluoropentanol or hexafluorobutanol.

The C₁₋₈ ketones are selected from acetone, methylisobutylketone, methylethylketone, or 3-hydroxy-3-methyl-2-butanone.
The halogen substituted C₁₋₄ alkanes are selected from chloroform, dichloromethane or 1-chlorobutane.
The amides are selected from dimethylformamide or dimethylacetamide.

Finally the optical layer (dye layer) can be coated by a further optical layer, by a protective layer, by an adhesive layer or by a reflective layer.

### Examples

All compounds are prepared using standard procedures known in the art. The precipitate is isolated following standard methods.

### Synthesis of 2-(1,1-Dioxo-1,2-dihydro-1-lambda-6-benzo[b]thiophen-3-ylidene)-malononitrile

20 Parts 3-oxo-2,3-dihydro-1-benzothiophene-1,1-dioxide, 8 parts malonic acid dinitrile and 0.2 parts of a mixture (1:5) of piperidine and glacial acetic acid are dissolved in 250 parts anhydrous ethanol and heated to 60°C for 6 hours with stirring. A further 4 parts of malonic acid dinitrile are added and the mixture is stirred further for 16 hours at 60°C. Subsequently, the mixture is cooled to 10 to 5°C and the product of the following formula (II) is filtered, washed with ice-cold ethanol and dried in vacuo.

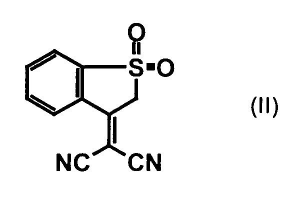

### Example 1

12 Parts 3-N,N-di-n-hexyl-toluidine are dissolved in 50 parts dry dimethylformamide, reacted with 7 parts phosphorous oxychloride under cooling and subsequently heated to 60°C for 4 hours. The cooled solution is added dropwise at room temperature with stirring to a suspension of 10 parts of compound (II) of above with 15 parts anhydrous sodium acetate in 200 parts absolute alcohol. The mixture is stirred over-night at room temperature and the precipitated dye of formula (III) is filtered, washed with ice-cold absolute alcohol, slurried in 500 parts ice water, filtered again, washed with ice-cold water and dried in vacuo. 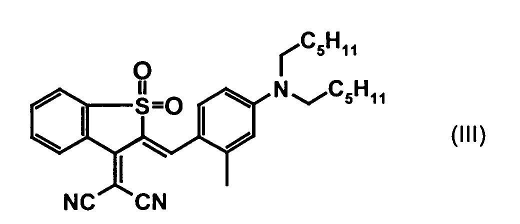 Yield : 74%; FT-IR (cm⁻¹): 2217, 1611, 1479, 1353, 1165, 1082; mp.: 220°C; UV-VIS (MeOH) *lambda* max : 608.5 nm; *epsilon* (*lambda* max) : 32000 l. mol⁻¹.cm⁻¹

### Example 2

13 parts of compound (II) of above and 10 parts of 4-pyrrolidino-benzaldehyde in 50 parts absolute alcohol are heated to reflux for 8 hours. The mixture is cooled to room temperature and the precipitated dye of formula (IV) is filtered, washed with ethyl acetate, water and then dried in vacuo. 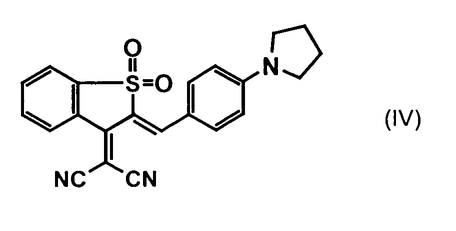 Yield : 87%; FT-IR (cm⁻¹): 2194, 1615, 1491, 1332, 1167, 824; mp.: 213°C; UV-VIS (MeOH) *lambda* max : 590.0 nm, *epsilon* (*lambda* max) : 23000 l. mol⁻¹.cm⁻¹

### Example 3

13 parts of compound (II) of above and 10 parts of 4-diethylamino-benzaldehyde in 50 parts absolute alcohol are heated to reflux for 8 hours. The mixture is cooled to room temperature and the precipitated dye of formula (V) is filtered, washed with ethyl acetate, water and then dried in vacuo. 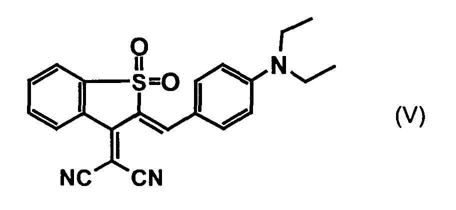 Yield : 81.3%; FT-IR (cm⁻¹): 2207, 1614, 1491, 1335, 1192, 900; mp.: 226°C; UV-Vis (MeOH) *lambda* max : 588 nm; *epsilon* (*lambda* max) : 32000 l. mol⁻¹.cm⁻¹

### Example 4

13 parts of compound (II) of above and 12 parts of julolidinecarbaldehyde in 50 parts absolute alcohol are heated to reflux for 8 hours. The mixture is cooled to room temperature and the precipitated dye of formula (VI) is filtered, washed with ethyl acetate, water and then dried in vacuo. 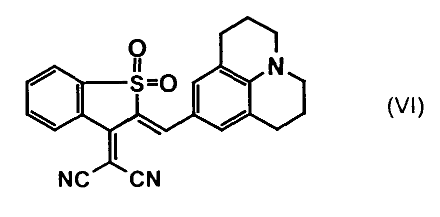 Yield : 90.2%; FT-IR (cm⁻¹): 2203, 1625, 1487, 1314, 1168, 922; mp.: 226°C; UV-VIS (MeOH) *lambda* max : 624 nm; *epsilon* (*lambda* max): 46000 l. mol⁻¹.cm⁻¹

### Example 5

13 parts of compound (II) of above and 10 parts of 4-diethylamino-2-hydroxybenzaldehyde in 50 parts absolute alcohol are heated to reflux for 8 hours. The mixture is cooled to room temperature and the precipitated dye of formula (VII) is filtered, washed with ethyl acetate, water and then dried in vacuo. 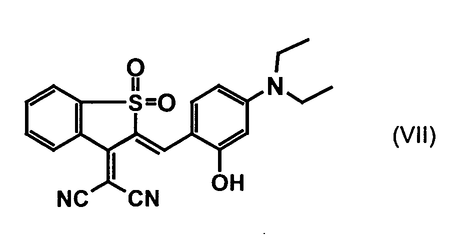 Yield : 85%; FT-IR (cm⁻¹): 2212, 1618, 1484, 1340, 1211, 924; mp.: 223°C; UV-VIS (MeOH) *lambda* max : 589.5 nm; *epsilon* (*lambda* max) : 49000 l. mol⁻¹.cm⁻¹

### Example 6

0.30 g of the dye of formula (III) obtained from example 1 were dissolved in 10 ml of tetrafluoropropane, and stirred at room temperature for 5 hours. The solution was filtered with a Teflon filter (having 0,2 µm pore size) and spin coated over a substrate to form a dye layer. The substrate was a polycarbonate substrate with 0.6 mm thickness having pregrooves with 150 nm depth, 300 nm width and 800 nm track pitch.
The substrate having the dye layer was dried in a vacuum oven set at 40°C for 12 hours. The optical layers obtained had a uniform and even surface at a thickness of 120 nm.
As a reflective layer, silver was deposited over the dye layer with a thickness of 100 nm. An acrylic UV curable resin was spin-coated over the reflective layer and then cured by radiation of UV rays to form a protective layer.

Finally, the substrate with the reflective layer and the recording layer was combined with another blank substrate with a thickness of 0.6 mm. The final product is a high-density recordable optical disc of about 120 mm diameter.

To evaluate the final product a PULSTEC DDU-1000 evaluation test machine was used to write and read the test results.

The recording conditions were: constant linear velocity (CLV) at 3.5 m/s, wavelength at 658 nm, numerical aperture (NA) at 0.6, and writing power from 7-14 mW.

The reading conditions were: CLV at 2.5 m/s, wavelength at 658nm, NA at 0.6, and reading power from 0.5 to 1.5 mW.

The tests provided for results within the expectations with regard to absorptivity, reflectance and recording sensitivity. Further, the obtained optical layers were sufficiently stable to light and heat. The optical recording media were storage stable within specification.

### Example 7-10

Optical recording media with an optical layer comprising the dyes obtained according to examples 2 to 5 (compounds of formula (IV)-(VII)) were manufactured and tested according to the procedure given for example 6.

## Claims

1. An optical layer comprising at least one blue disperse dye of the formula (I) 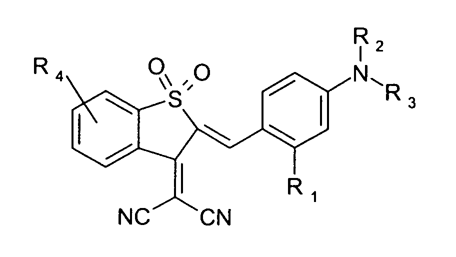 wherein
R₁ is selected from hydrogen, hydroxyl, C₁₋₈ alkyl, C₁₋₈ alkoxyalkyl, trifluoromethyl, carboxyl (COOH), carboxylic ester (COOR⁵) nitro (NO₂), amide (CONR⁶R⁷), sulfonate (SO₃R⁸), and halogen (-F, -Cl, -Br);
R² and R³ are independently selected from hydrogen, C₁₋₈ alkyl, C₁₋₈ alkoxyalkyl, cyanoalkyl, or
R² and R³ can be connected to form a pyrrolidine ring, a piperidine ring or a morpholine ring, or
R² and R³ can form a julolidine ring connecting R² and R³ to the ortho-positions at the benzene ring;
R₄ is selected from hydrogen, C₁₋₂ alkyl, C₁₋₂ alkoxyalkyl, nitro (NO₂), and halogen (-F, -Cl, -Br);
R⁵, R⁶, R⁷or R⁸ are selected from hydrogen and C₁₋₈ alkyl.

2. An optical layer according to claim 1, wherein
R₁ is selected from hydrogen, hydroxyl, C₁₋₂ alkyl, C₁₋₂ alkoxyalkyl, and halogen (-F, -Cl, -Br);
R² and R³ are independently selected from C₁₋₈ alkyl, C₁₋₈ alkoxyalkyl, or
R² and R³ can be connected to form a pyrrolidine ring, or
R² and R³ can form a julolidine ring connecting R² and R³ to the ortho-positions at the benzene ring;
R₄ is selected from hydrogen, C₁₋₂ alkyl, C₁₋₂ alkoxyalkyl, nitro (NO₂), and halogen (-F, -Cl, -Br).

3. An optical layer according to claim 1, wherein
R₁ is selected from Methyl, Methoxy, Chloro or Bromo;
R² and R³ are independently selected from C₂₋₆ alkyl, or
R² and R³ can be connected to form a pyrrolidine ring, or
R² and R³ can form a julolidine ring connecting R² and R³ to the ortho-positions at the benzene ring;
R₄ is hydrogen.

4. An optical layer according to claim 1, wherein
R₁ is methyl,
R₂ and R₃ are n-Hexyl, and
R₄ is hydrogen.

5. A method for producing optical layers according to claims 1 to 4, comprising the following steps
(a) providing a substrate
(b) dissolving a compound of formula (I) as defined in claims 1 to 4 in an organic solvent to form a solution,
(c) coating the solution (b) on the substrate (a);
(d) evaporating the solvent to form a dye layer.

6. A method according to claim 5, wherein the organic solvent is selected from C₁₋₈ alcohol , halogen substituted C₁₋₈ alcohols, C₁₋₈ ketone, C₁₋₈ ether, halogen substituted C₁₋₄ alkane, or amides.

7. A method according to claim 6, wherein the C₁₋₈ alcohols or halogen substituted C₁₋₈ alcohols are selected from methanol, ethanol, isopropanol, diacetone alcohol (DAA), 2,2,3,3-tetrafluoropropanol, trichloroethanol, 2-chloroethanol, octafluoropentanol or hexafluorobutanol;
the C₁₋₈ ketones are selected from acetone, methylisobutylketone, methylethylketone, or 3-hydroxy-3-methyl-2-butanone;
the halogen substituted C₁₋₄ alkanes are selected from chloroform, dichloromethane or 1-chlorobutane; and
the amides are selected from dimethylformamide or dimethylacetamide.

8. A method according to claim 5, wherein the substrate is polycarbonate (PC) or polymethylmethacrylate (PMMA).

9. A method according to claim 5, wherein the optical layer (dye layer) has a thickness from 70 to 250 nm.

10. An optical recording medium comprising an optical layer according to claims 1 to 4, wherein at least one blue disperse dye of the formula (I) as defined in claims 1 to 4 is used.
